# FASCICULE DE BREVET EUROPEEN

(11) **EP 3 442 633 B1**
(45) Date de publication et mention de la délivrance du brevet: **05.02.2020**
(21) Numéro de dépôt: 17723448.1
(22) Date de dépôt: 13.04.2017
(51) Int. Cl.: A61M 16/00, A61M 15/00

(54) **DISPOSITIF DE DISTRIBUTION DE PRODUIT FLUIDE SYNCHRONISE AVEC L'INHALATION**
VORRICHTUNG ZUR AUSGABE EINES MIT DER INHALATION SYNCHRONISIERTEN FLUIDPRODUKTS
DEVICE FOR DISPENSING A FLUID PRODUCT SYNCHRONISED WITH INHALATION

(30) Priorité: 15.04.2016 FR 1653367
(43) Date de publication de la demande: 20.02.2019
(73) Titulaire: Aptar France SAS, 27110 Le Neubourg (FR)
(72) Inventeur: PETIT, Ludovic, 27110 Vitot (FR)
(74) Mandataire: CAPRI
(86) Numéro de dépôt international: PCT/FR2017/050895
(87) Numéro de publication internationale: WO 2017/178768

(56) Documents cités:
- EP-A1- 0 441 643
- WO-A1-85/01880
- DE-A1- 3 040 641
- FR-A1- 2 775 668
- US-A- 5 060 643

## Description

La présente invention concerne un dispositif de distribution de produit fluide synchronisé avec l'inhalation, et plus particulièrement un dispositif d'inhalation du type aérosol synchronisé avec l'inhalation.

Les dispositifs actionnés par l'inhalation, désignés généralement par le terme B.A.I. (signifiant "Breath Actuated Inhaler"), sont bien connus dans l'état de la technique. L'avantage principal de ce type de dispositif est que la distribution du produit est synchronisée avec l'inhalation du patient, pour garantir une bonne distribution du produit dans les voies respiratoires. Ainsi, dans le domaine des dispositifs aérosol, c'est-à-dire ceux dans lesquels le produit est distribué à l'aide d'un gaz propulseur, de nombreux types de dispositifs de déclenchement par l'inhalation ont été proposés. Ces dispositifs présentent toutefois l'inconvénient de comporter un grand nombre de pièces, c'est-à-dire qu'ils sont compliqués et coûteux à fabriquer et à assembler, ce qui est évidemment désavantageux. Il est aussi difficile de trouver le bon équilibre entre un déclenchement fiable à chaque inhalation, sans que le seuil de déclenchement ne soit trop élevé, et une serrure suffisamment robuste pour empêcher un actionnement accidentel ou non souhaité. Or, si la serrure se déverrouille de manière accidentelle, le dispositif est actionné automatiquement et la dose est distribuée, même si l'utilisateur ne l'a pas souhaité.

Ainsi, plus que l'actionnement automatique du dispositif, ce qui est important pour obtenir une bonne distribution de la dose, c'est de réaliser cette distribution de manière synchronisée avec l'inhalation de l'utilisateur, même si l'actionnement ou le déclenchement proprement dit reste manuel.

Le document FR2775668 décrit un dispositif de l'état de la technique.

La présente invention a pour but de fournir un dispositif de distribution de produit fluide synchronisé avec l'inhalation qui ne reproduit pas les inconvénients susmentionnés.

La présente invention a aussi pour but de fournir un dispositif de distribution de produit fluide synchronisé avec l'inhalation qui améliore la fiabilité de fonctionnement, en garantissant un actionnement efficace à chaque inhalation.

La présente invention a aussi pour but de fournir un dispositif de distribution de produit fluide synchronisé avec l'inhalation qui minimise les risques d'actionnement accidentel ou non souhaité.

La présente invention a également pour but de fournir un dispositif de distribution de produit fluide synchronisé avec l'inhalation qui ne présente pas un seuil de déclenchement trop élevé, permettant ainsi à des personnes relativement faibles, telles que des personnes malades ou âgées, d'utiliser le dispositif de manière sûre et fiable.

La présente invention a également pour but de fournir un dispositif de distribution de produit fluide synchronisé avec l'inhalation qui soit simple et peu coûteux à fabriquer et à assembler.

La présente a donc pour objet un dispositif de distribution de produit fluide synchronisé avec l'inhalation, comportant un corps pourvu d'un embout buccal, un réservoir de produit contenant un produit fluide et un gaz propulseur étant monté axialement coulissant dans ledit corps, une valve doseuse comportant une soupape étant assemblée sur ledit réservoir pour distribuer sélectivement le produit fluide, ledit dispositif comportant:
- un élément d'actionnement déplaçable et/ou déformable entre une position de non actionnement, dans laquelle ladite valve doseuse ne peut pas être actionnée, et une position d'actionnement, dans laquelle ladite valve doseuse peut être actionnée, et
- un système de déclenchement commandé par l'inhalation comportant un organe sensible à l'inhalation, déformable et/ou déplaçable sous l'effet de l'inhalation, ledit organe sensible à l'inhalation, lorsqu'il se déforme et/ou se déplace, déplaçant et/ou déformant ledit élément d'actionnement de sa position de non actionnement vers sa position d'actionnement, ledit élément d'actionnement étant un élément de verrouillage qui, en position de non actionnement, permet le déplacement axial de ladite soupape de la valve doseuse ensemble avec ledit réservoir dans le corps, empêchant l'actionnement de ladite valve doseuse lorsque ledit réservoir est déplacé axialement dans le corps sans inhalation.

Avantageusement, lors de l'inhalation, ledit élément de verrouillage est déplacé et/ou déformé de telle sorte qu'il bloque le déplacement axial de la soupape par rapport au corps.

Avantageusement, ledit système de déclenchement commandé par l'inhalation comporte un piston coulissant dans une chambre entre une position de repos et une position d'inhalation.

Avantageusement, ledit élément de verrouillage est solidaire d'une tige solidaire du piston, de sorte que lors de l'inhalation, ladite tige se déplace radialement déplaçant ledit élément de verrouillage vers sa position d'actionnement dans laquelle il empêche le déplacement axial de ladite soupape de la valve doseuse lorsque ledit réservoir est déplacé axialement dans le corps.

Avantageusement, ledit dispositif comporte un compteur de doses électronique.

Avantageusement, ledit dispositif comporte des moyens d'émission de signaux pour communiquer à distance notamment des informations relatives aux actionnements du dispositif.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en section d'un dispositif de distribution de produit fluide, selon un mode de réalisation avantageux, en position de repos,
- la figure 2 est une vue similaire à celle de la figure 1, lorsque l'utilisateur tente d'actionner le dispositif sans inhalation, et
- la figure 3 est une vue similaire à celle de la figure 2, lorsque l'utilisateur actionne le dispositif avec inhalation simultanée.

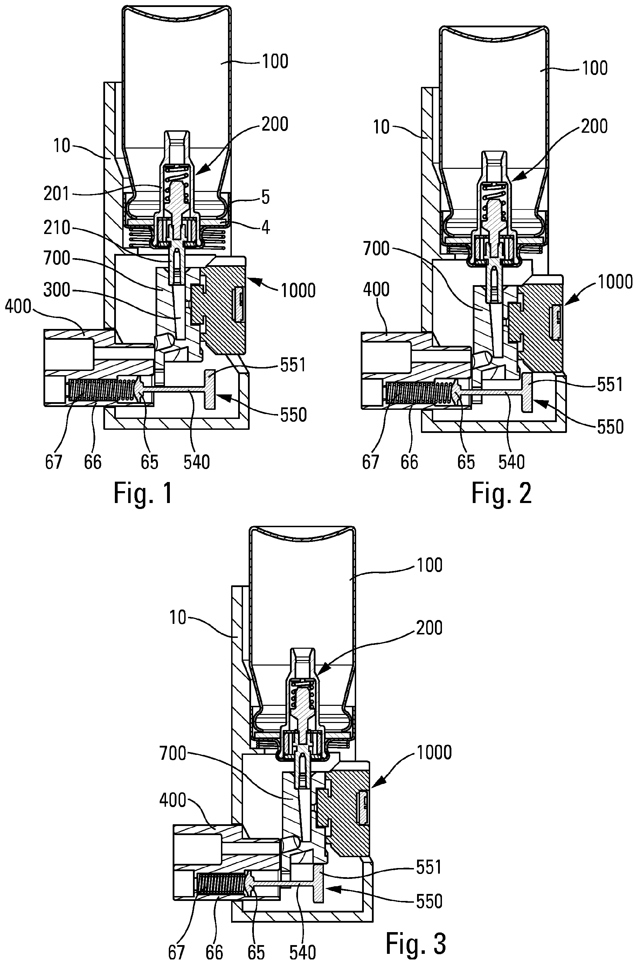

Dans la description, les termes "supérieur", "inférieur", "haut" et "bas" se réfèrent à la position du dispositif représentée notamment sur la figure 1. Les termes "axial" et "radial" se réfèrent à l'axe central vertical. Les termes "proximal" et "distal" se réfèrent à l'embout buccal.

L'invention s'applique plus particulièrement à des dispositifs d'inhalation du type à valve aérosol pour une distribution orale, comme cela sera décrit plus en détail ci-après, mais elle pourrait aussi s'appliquer à d'autres types de dispositifs d'inhalation, par exemple du type nasal.

Les figures représentent un mode de réalisation avantageux de l'invention, mais il est entendu que l'une ou plusieurs des pièces constitutives décrites ci-après pourrai(en)t être réalisée(s) différemment tout en procurant des fonctions similaires voire identiques.

En référence aux dessins, le dispositif comporte un corps principal 10 pourvu d'un embout buccal 400. Cet embout buccal 400 définit un orifice de distribution à travers lequel l'utilisateur va inhaler lors de l'utilisation du dispositif. Un capot de protection amovible peut être prévu sur ledit embout buccal 400, notamment lors des périodes de stockage, que l'utilisateur va retirer avant utilisation. Le corps 10 contient un réservoir 100, contenant le produit à distribuer et un gaz propulseur, tel qu'un gaz du type HFA, une valve doseuse 200 étant montée sur ledit réservoir 100 pour distribuer sélectivement le produit. La valve doseuse 200 comporte un corps de valve 201 et une soupape 210 axialement déplaçable lors de l'actionnement par rapport audit corps de valve 201, et donc par rapport audit réservoir 100. Cette valve doseuse 200 peut être d'un type quelconque approprié. Elle peut être fixée au réservoir 100 par un élément de fixation, de préférence une capsule sertie 5, de préférence avec interposition d'un joint de col 4.

Avantageusement, lors de l'actionnement, la soupape 210 est fixe par rapport au corps 10, et c'est le réservoir 100 qui est déplacé axialement par rapport au corps 10 entre une position distale, qui est la position de repos, et une position proximale.

L'orifice de sortie de la soupape 210 de ladite valve doseuse 200 est relié via un canal 300 audit embout buccal 400, à travers lequel l'utilisateur inhale le produit distribué. De manière connue, ladite soupape 210 est reçue dans un puits de soupape 700 qui définit au moins partiellement ledit canal 300. Ledit puits de soupape est axialement déplaçable par rapport audit corps 10.

Selon l'invention, le dispositif comporte un élément d'actionnement 550 déplaçable et/ou déformable entre une position de non actionnement, dans laquelle ladite valve doseuse 200 ne peut pas être actionnée, et une position d'actionnement, dans laquelle ladite valve doseuse 200 peut être actionnée. En position de repos, ledit élément d'actionnement 550 est en position de non actionnement, et c'est l'inhalation de l'utilisateur à travers l'embout buccal 400 qui déplace et/ou déforme ledit élément d'actionnement 550 vers sa position d'actionnement. En d'autres termes, tant que l'utilisateur n'a pas inhalé, l'actionnement de la valve doseuse 200 est impossible, et ce n'est que lorsqu'il inhale qu'il peut actionner ladite valve doseuse 200, avantageusement par appui manuel sur le fond du réservoir 100.

Selon l'invention, l'élément d'actionnement est un élément de verrouillage 550 qui, en position de non actionnement, permet le déplacement axial de la soupape 210 de la valve doseuse 200 ensemble avec réservoir 100 dans le corps 10, empêchant par-là l'actionnement de ladite valve doseuse 200 lorsque ledit réservoir 100 est déplacé axialement dans le corps 10 sans inhalation. Lors de l'inhalation, cet élément de verrouillage 550 est déplacé et/ou déformé de telle sorte qu'il bloque le déplacement axial de la soupape 210 par rapport au corps 10. Ainsi, après inhalation, un déplacement axial du réservoir 100 provoque l'actionnement de la valve doseuse 200 et la distribution d'une dose de produit synchronisée avec cette inhalation.

Ainsi, en l'absence d'inhalation, il n'y a aucun risque qu'une dose de produit actif soit perdue par un actionnement malencontreux ou incomplet dans lequel l'utilisateur n'exercerait pas d'inhalation. L'actionnement de la valve 200 et l'expulsion d'une dose de produit fluide ne sont donc possibles que si l'utilisateur inhale et que simultanément il appuie sur le réservoir 100 pour actionner la valve 200.

Le dispositif comporte un système de déclenchement commandé par l'inhalation de l'utilisateur, qui est destiné à déplacer et/ou déformer ledit élément d'actionnement 550 de sa position de non actionnement vers sa position d'actionnement, lorsque l'utilisateur inhale à travers l'embout buccal 400.

Ce système de déclenchement comporte un organe sensible à l'inhalation 65, déformable et/ou déplaçable sous l'effet de l'inhalation, cet organe sensible à l'inhalation 65 étant adapté, lorsqu'il se déforme et/ou se déplace, à déplacer et/ou déformer ledit élément d'actionnement 550 de sa position de non actionnement vers sa position d'actionnement.

Comme cela sera décrit plus en détails ci-après, l'organe sensible à l'inhalation peut être réalisé sous la forme d'un piston 65, de préférence cylindrique, coulissant dans une chambre 66, de préférence cylindrique, non déformable.

En variante, l'organe sensible à l'inhalation pourrait aussi être réalisé sous la forme d'une chambre d'air déformable, par exemple un soufflet ou une poche déformable.

Les figures 1 à 3 illustrent un mode de réalisation de l'invention. L'élément d'actionnement est un élément de verrouillage 550 qui, en position de non actionnement, permet le déplacement axial de la soupape 210 de la valve doseuse 200 ensemble avec le réservoir 100 dans le corps 10, empêchant l'actionnement de ladite valve doseuse 200 lorsque ledit réservoir 100 est déplacé axialement dans le corps 10 sans inhalation. Lors de l'inhalation, cet élément de verrouillage 550 est déplacé et/ou déformé de telle sorte qu'il bloque le déplacement axial de la soupape 210 par rapport au corps 10. Ainsi, après inhalation, un déplacement axial du réservoir 100 provoque l'actionnement de la valve doseuse 200 et la distribution d'une dose de produit synchronisée avec cette inhalation.

L'organe sensible à l'inhalation est réalisé sous la forme d'un piston 65 coulissant dans une chambre 66, entre une position de repos et une position d'inhalation. La chambre 66 est avantageusement formée dans l'embout buccal 400. Ledit piston 65 est relié audit élément de verrouillage 550, avantageusement par une tige 540. En particulier, comme visible sur les figures, l'élément de verrouillage 550 est formé à l'extrémité de ladite tige 540 opposée audit piston 65, et comprend une projection axiale 551. Un ressort 67, disposé avantageusement dans la chambre 66, est adapté à ramener ledit piston 65 vers sa position de repos lorsqu'il n'y a plus d'inhalation à travers l'embout buccal 400.

En position de non actionnement, ladite projection est décalée radialement par rapport au puits de soupape 700, de sorte que celui-ci peut se déplacer axialement dans le corps 10, ensemble avec la soupape 210 de la valve doseuse 200 et le réservoir 100. Ainsi, dans cette position de non actionnement, la soupape 210 ne se déplace par rapport au réservoir 100, et la valve doseuse 200 n'est donc pas actionnée.

Lorsque l'utilisateur inhale à travers l'embout buccal 400, le piston 65 se déplace radialement (par rapport à l'axe de déplacement du réservoir 100 dans le corps 10) dans la chambre 66 sous l'effet de la dépression crée par l'inhalation. La projection 551 se déplace donc également radialement, et vient se positionner sous ledit puits de soupape 700, formant ainsi une butée au déplacement axial vers le bas dudit puits de soupape. De ce fait, la pression exercée par l'utilisateur sur le fond du réservoir 100 va déplacer celui-ci axialement vers le bas dans le corps, et le puits de soupape 700, maintenant axialement fixe par rapport au corps 10, va donc bloquer la soupape 210 de la valve doseuse axialement par rapport au corps 10, de sorte qu'elle va s'enfoncer dans le corps de valve, provoquant ainsi l'actionnement de la valve doseuse 200 et la distribution d'une dose de produit fluide.

Le dispositif représenté sur les figures 1 à 3 peut comprendre également des moyens électroniques En particulier, il peut être prévu un compteur de doses électronique 1000, avantageusement assemblé sur le corps 10. Bien entendu, le réservoir 100 étant déplaçable axialement dans le corps 10 aussi bien en position d'actionnement que de non actionnement de l'élément d'actionnement 550, le compteur de dose 1000 ne peut pas mesurer ce déplacement axial du réservoir 100. On privilégiera dans ce cas des capteurs détectant la distribution du produit fluide, notamment dans le puits de soupape 700, ou des capteurs détectant le déplacement de la soupape 210 de la valve doseuse 200 par rapport au corps de valve 201.

De préférence, le dispositif comporte aussi des moyens d'émission de signaux 1100 pour communiquer à distance notamment des informations relatives aux actionnements du dispositif. En particulier, le corps 10 peut comporter un module d'émission de signaux, pour communiquer à distance avec une base quelconque. Des moyens d'alimentation appropriés sont avantageusement prévus.

Avantageusement, le module électronique peut comprendre notamment une carte comportant un commutateur électrique qui envoie une impulsion. Le module peut également comporter un afficheur et/ou utiliser une connexion Bluetooth ou Wifi pour envoyer les informations sur un périphérique annexe. Des capteurs appropriés peuvent être prévus, tel que des capteurs de débit et/ou de pression, pour détecter divers paramètres du flux d'inhalation.

Associés à un compteur de dose 1000 qui compte chaque dose effectivement émise et au dispositif de synchronisation avec l'inhalation de l'invention, ces moyens d'émission de signaux 1100 permettent de transmettre de manière absolument fiable chaque distribution de dose, par exemple à un médecin ou toute autre personne souhaitant surveiller l'utilisation du dispositif d'inhalation par l'utilisateur. Le dispositif de synchronisation avec l'inhalation garantit que l'utilisateur inhale à chaque fois qu'il actionne le dispositif, et le compteur enregistre chaque dose distribuée, ainsi que différents paramètres associés, tel que l'horodatage de chaque distribution. Le médecin peut ainsi connaitre très exactement les conditions d'utilisation du dispositif par l'utilisateur.

La présente invention est notamment applicable pour le traitement des crises d'asthme ou de BPCO (Broncho Pneumopathie Obstructive) en utilisation avec des formulations de type salbutamol, aclidinium, formoterol, tiotropium, budesonide, fluticasone, indacaterol, glycopyrronium, salmeterol, umeclidinium bromide, vilanterol, olodaterol, striverdi, ou toute combinaison de ces formulations.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide synchronisé avec l'inhalation, comportant un corps (10) pourvu d'un embout buccal (400), un réservoir de produit (100) contenant un produit fluide et un gaz propulseur étant monté axialement coulissant dans ledit corps (10), une valve doseuse (200) comportant une soupape (210) étant assemblée sur ledit réservoir (100) pour distribuer sélectivement le produit fluide, ledit dispositif comportant:
- un élément d'actionnement (550) déplaçable et/ou déformable entre une position de non actionnement, dans laquelle ladite valve doseuse (200) ne peut pas être actionnée, et une position d'actionnement, dans laquelle ladite valve doseuse (200) peut être actionnée, et
- un système de déclenchement commandé par l'inhalation comportant un organe sensible à l'inhalation (61; 65), déformable et/ou déplaçable sous l'effet de l'inhalation, ledit organe sensible à l'inhalation (61; 65), lorsqu'il se déforme et/ou se déplace, déplaçant et/ou déformant ledit élément d'actionnement (550) de sa position de non actionnement vers sa position d'actionnement,
**caractérisé en ce que** ledit élément d'actionnement est un élément de verrouillage (550) qui, en position de non actionnement, permet le déplacement axial de ladite soupape (210) de la valve doseuse (200) ensemble avec ledit réservoir (100) dans le corps (10), empêchant l'actionnement de ladite valve doseuse (200) lorsque ledit réservoir (100) est déplacé axialement dans le corps (10) sans inhalation.

2. Dispositif selon la revendication 1, dans lequel, lors de l'inhalation, ledit élément de verrouillage (550) est déplacé et/ou déformé de telle sorte qu'il bloque le déplacement axial de la soupape (210) par rapport au corps (10).

3. Dispositif selon la revendication 2, dans lequel ledit système de déclenchement commandé par l'inhalation comporte un piston (65) coulissant dans une chambre (66) entre une position de repos et une position d'inhalation.

4. Dispositif selon la revendication 3, dans lequel ledit élément de verrouillage (550) est solidaire d'une tige (540) solidaire du piston (65), de sorte que lors de l'inhalation, ladite tige (540) se déplace radialement déplaçant ledit élément de verrouillage (550) vers sa position d'actionnement dans laquelle il empêche le déplacement axial de ladite soupape (210) de la valve doseuse (200) lorsque ledit réservoir (100) est déplacé axialement dans le corps (10).

5. Dispositif selon l'une quelconque des revendications précédentes, comportant un compteur de doses électronique (1000).

6. Dispositif selon l'une quelconque des revendications précédentes, comportant des moyens d'émission de signaux (1100) pour communiquer à distance notamment des informations relatives aux actionnements du dispositif.

## Patentansprüche

1. Vorrichtung zur inhalationssynchronisierten Ausgabe eines flüssigen Produkts, umfassend einen Körper (10), der mit einem Mundstück (400) versehen ist, einen Produkttank (100), der ein flüssiges Produkt und ein Treibgas enthält, und der axial verschiebbar in dem Körper (10) angebracht ist, ein Dosierventil (200), umfassend ein Ventil (210), das auf dem Tank (100) befestigt ist, um das flüssige Produkt selektiv abzugeben, wobei die Vorrichtung umfasst:
- ein Betätigungselement (500), das zwischen einer Nichtbetätigungsposition bewegbar und/oder verformbar ist, in der das Dosierventil (200) nicht betätigbar ist, und einer Betätigungsposition, in der das Dosierventil (200) betätigbar ist, und
- ein inhalationsgesteuertes Auslösesystem, umfassend ein inhalationsempfindliches Element (61; 65), das durch die Inhalation verformbar und/oder bewegbar ist, wobei das inhalationsempfindliche Element (61; 65) bei Verformung und/oder Bewegung das Betätigungselement (500) aus seiner Nichtbetätigungsposition in seine Betätigungsposition bewegt und/oder verformt,
**dadurch gekennzeichnet, dass** das Betätigungselement ein Verriegelungselement (550) ist, das, in der Nichtbetätigungsposition, die axiale Bewegung des Ventils (210) des Dosierventils (200) zusammen mit dem Tank (100) in dem Körper (10) ermöglicht, wobei es die Betätigung des Dosierventils (200) verhindert, wenn der Tank (100) in dem Körper (10) ohne Inhalation axial bewegt wird.

2. Vorrichtung nach Anspruch 1, wobei das Verriegelungselement (550) während der Inhalation derart bewegt und/oder verformt wird, dass es die axiale Bewegung des Ventils (210) in Bezug auf den Körper (10) blockiert.

3. Vorrichtung nach Anspruch 2, wobei das inhalationsgesteuerte Auslösesystem einen Kolben (65) umfasst, der in einer Kammer (66) zwischen einer Ruheposition und einer Inhalationsposition verschiebbar ist.

4. Vorrichtung nach Anspruch 3, wobei das Verriegelungselement (550) an einer Stange (540) befestigt ist, die an dem Kolben (65) befestigt ist, so dass sich die Stange (540) während der Inhalation radial bewegt, wobei das Verriegelungselement (550) in seine Betätigungsposition bewegt wird, in der es verhindert, dass das Ventil (210) des Dosierventils (200) axial bewegt wird, wenn der Tank (100) axial in dem Körper (10) bewegt wird.

5. Vorrichtung nach einem der vorhergehenden Ansprüche, umfassend einen elektronischen Dosiszähler (1000).

6. Vorrichtung nach einem der vorhergehenden Ansprüche, umfassend Signalübertragungseinrichtungen (1100) zum Fernübermitteln insbesondere von Informationen, die sich auf die Betätigungen der Vorrichtung beziehen.

## Claims

1. An inhalation-synchronized fluid dispenser device comprising a body (10) provided with a mouthpiece (400), a fluid reservoir (100) containing a fluid and a propellant gas being mounted to slide axially in said body (10), a metering valve (200) including a valve member (210) being assembled on said reservoir (100) for selectively dispensing the fluid, said device further comprising:
- an actuator element (550) that is movable and/or deformable between a non-actuation position, in which said metering valve (200) cannot be actuated, and an actuation position, in which said metering valve (200) can be actuated, and
- an inhalation-controlled trigger system including an inhalation-sensitive member (61; 65), deformable and/or movable under the effect of inhaling, said inhalation-sensitive member (61; 65), when it is deformed and/or moved, moving and/or deforming said actuator element (550) from its non-actuation position towards its actuation position,
**characterized in that** said actuator element is a locking element (550) that, in non-actuation position, enables axial movement of said valve member (210) of the metering valve (200) together with said reservoir (100) in the body (10), preventing the actuation of said metering valve (200) when said reservoir (100) is moved axially in the body (10) without inhaling.

2. A device according to claim 1, wherein, during inhaling, said locking element (550) is moved and/or deformed so that it prevents the valve member (210) from moving axially relative to the body (10).

3. A device according to claim 2, wherein said inhalation-controlled trigger system includes a piston (65) that slides in a chamber (66) between a rest position and an inhaling position.

4. A device according to claim 3, wherein said locking element (550) is secured to a rod (540) secured to the piston (65), so that during inhaling, said rod (540) moves radially moving said locking element (550) towards its actuation position in which it prevents said valve member (210) of the metering valve (200) from moving axially when said reservoir (100) is moved axially in the body (10).

5. A device according to any preceding claim, including an electronic dose counter (1000).

6. A device according to any preceding claim, including signal-transmitter means (1100) for communicating remotely in particular information relating to the actuations of the device.
